# EUROPEAN PATENT APPLICATION

(11) **EP 0 887 472 A1**
(43) Date of publication of application: **30.12.1998**
(21) Application number: 96942662.6
(22) Date of filing: 27.12.1996
(51) Int. Cl.: E02B 5/02, E02B 3/14

(54) **TREE PLANTING CONCRETE PRODUCT**

(71) Applicant: Hirano, Norihiko, Gunma 371 (JP)
(72) Inventor: Hirano, Norihiko, Gunma 371 (JP)
(74) Representative: Leale, Robin George
(86) International application number: JP9603848
(87) International publication number: WO9829612

(57) **Abstract**

A tree planting concrete product in which carbon fiber strands are planted in a predetermined position of an outer surface of a concrete product body used in an environment in which the water, the amount of which is required to germinate seeds, is supplied, characterized in that the concrete product body is provided with strand planatation holes, in which locking members are inserted and fixed, one end portion of each of the carbon fiber strand groups being gripped by one of the locking members.

## Description

### TECHNICAL FIELD

The present invention relates to concrete product for afforestation suitable for growing microorganisms and plants. More particularly, the invention relates to concrete products provided with green-compatible processing to the concrete secondary products such as U-shaped blocks to be used in water channels, and square blocks which are used for levee of river banks and sea coast, by planting carbon fiber strands made by bundling carbon fibers.

### BACKGROUND ART

In recent years, because the banks of rivers, coastal walls and the like which are constructed by concrete structures provide inorganic landscape, social requests for covering the concrete structures with green have increased.

Also, the fact that the natural riverside zones in which the aquatic plants had grown came to be covered with levee walls means to cause loss of aquatic purification function to provide a cause for contamination of river water.

Furthermore, the rivers which have been provided with levee works linearly and plainly by concrete structures have lost variable streams such as pools, falling sports, shoal, etc, which are observed with natural rivers, with the result that there have been provided environments suitable for habitation of the aquatic livings.

In order to solve the problems as above, there have been developed techniques on concrete products incorporated with the structures useful for growing microorganisms and plants, which are for example the first technique as shown in Fig. 9 (Japanese Utility Model Publication No. Hei-4-39862), the second technique as shown in Fig. 10 (Japanese Laid-open Patent No. Sho-62-280425), the third technique as shown in Fig. 11 (PCT/JP95/02008), and the like.

The first technique denotes a structure made by imbedding or mixing in carbonaceous matters 30 provided with activated carbon properties in the concrete block body 29, having constitution of combining a porous concrete wall material 31 with said structure.

The second technique relates to the green-compatible square constructing method and square construction blocks which are characterized by laying in multi-layers and solidifying the square structure co-blocks 33 laminated with non-woven fabric mat-like shaped object 32 with mixed kneads of plant seeds, soil, water-retaining material, etc. 34 integrally.

However, these techniques require the work of previously filling the carbonaceous matter 30 or kneads 34 in the concrete products, and complicated structures, so that they have been unsuitable for practical application.

In view of the above, it has been provided a concrete product for acceleration of green growing which is the third technique of integrally planting carbon fiber strands 4 on the concrete block body 35 by burying the carbon fiber strands 4 in the concrete product. By this technique, without the necessity to fill or plant seeds of plant in the concrete product, because the living things settle on the carbon fiber strand 4 per se, green growing can be realized by natural force, so that contribution to water purification has been made.

However, the third technique has had the following problematic points:
Firstly, it is necessary to pour concrete in the frame under the state where the carbon fiber 4 is previously positioned in the frame. Therefore, the workability is very poor.
Secondly, because of integration by solidification of concrete, it is difficult to extract carbon fiber strands 4 after planting, and it is not possible to re-plant carbon fiber strands 4 or to adjust the number of the planted carbon fiber strands 4 to meet the environmental change such as place of installation at a later stage.

Furthermore, thirdly, in the work of replacing the carbon fiber strands according to necessity, replacement should be made in the unit of concrete block, which is inconvenient.

The present invention has been made in consideration of the problematic points of the conventional technique, and its object is to provide a concrete product for afforestations in which carbon fiber strands can be simply planted.

### DISCLOSURE OF THE INVENTION

In order to attain the above object, the present invention adopts the following means.

According to claim 1, there is provided a concrete product for afforestation, comprising carbon fiber strands planted in the predetermined position on the outer space of the concrete product body which is to be used under the environment in which sufficient amount of water necessary for germination of seeds is supplied. Said concrete product body has strand planting holes, and, by the flexible stopping member having a cross-sectional shape of C including an opening for introducing the strand on the side in which plant is inserted and fixed said strand planting hole, an end of said carbon fiber strand is held.

Since the flexible stopping member of C cross-sectional shape has adjustable aperture width of its side surface and adjustable outer diameter, a carbon fiber strand can be engaged with the central strand holding part from the side opening part, and a stopping member engaging the strand can be inserted in the strand planting hole.

According to claim 2, there is disclosed a concrete product for afforestation means according to claim 1, wherein, in said stopping member, an outer cylindrical part elastically engaging a strand planting hole and an inner cylindrical part which is a strand holding part are formed in one piece.

The outer cylindrical part acts to fix the stopping members to the plantation holes under pressure contact by the elasticity. Also, the inner cylindrical part is compressed in a direction of shortening both the outer cylinder and the inner cylinder to act to hold the strand for fixation. And, the space between the inner and the outer cylinders acts as a flow path of water to float the strand.

According to claim 3, there is disclosed a concrete product for afforestation means according to claim 2, wherein the above strand planting hole is formed in tapered shape so as to have the smaller diameter toward the exposed side of the carbon fiber strand, and the outer cylindrical part of the stopping member is formed in a tapered shape to meet the strand planting hole, and a stop-gap rib to be engaged with the peripheral part of the strand planting hole is formed at the tip of the outer cylindrical part.

Because of the formation of the strand planting hole and the outer cylindrical part in tapered form, when the stopping member is hit into the planting hole, the stopgap rib is engaged with the peripheral part of the hole, and the stopping member is fixed with stopgap to the hole.

According to claim 4, there is disclosed a concrete product for afforestation means according to any one of claims 1 to 3, wherein said concrete product has U-shaped grooves, and inside the side wall of the U-shaped groove body there are provided in projection the bulkheads for flowing water in zigzag directions right and left alternatively, and a strand planting hole provided on the respective bulkhead.

The bulkhead acts to reinforce the strength of the U-shaped groove and acts as a wall for forming strand planting hole. Also, the bulkhead projects into the water stream to act to change the flow and increase the dissolved oxygen amount in flowing water.

According to claim 5, there is provided a concrete product for afforestation means according to any one of claims 1 to 3, wherein the concrete product is a square faced block, with the upright wall projected on the front face of the square block body, and a planting hole provided on said upright wall.

The upright wall acts as a wall for forming strand planting hole, and projects into water stream to change the flow and acts to increase the dissolved oxygen amount in flowing water.

According to claim 5, there is provided a concrete product for afforestation means according to any one of claim 1 to 3, wherein the concrete product is a square block, and in front of the square block body a standing wall is provided in projection, and a strand planting hole is provided in said standing wall.

The standing wall acts as a wall for forming a hole for planting strand, and shows action to change water flow to increase the dissolved oxygen amount in water stream.

### BRIEF DESCRIPTION OF FIGURES

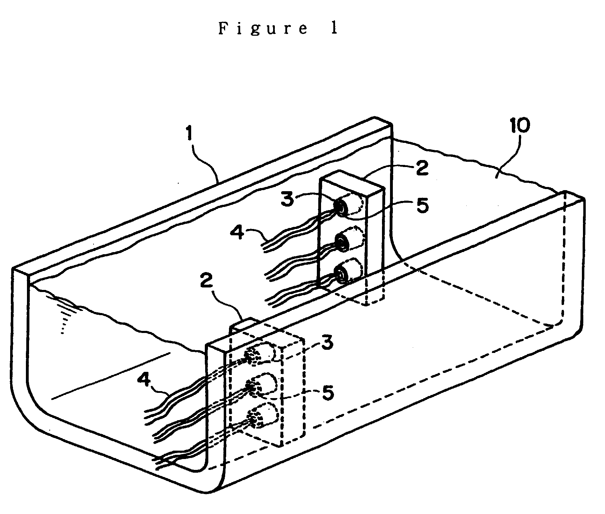

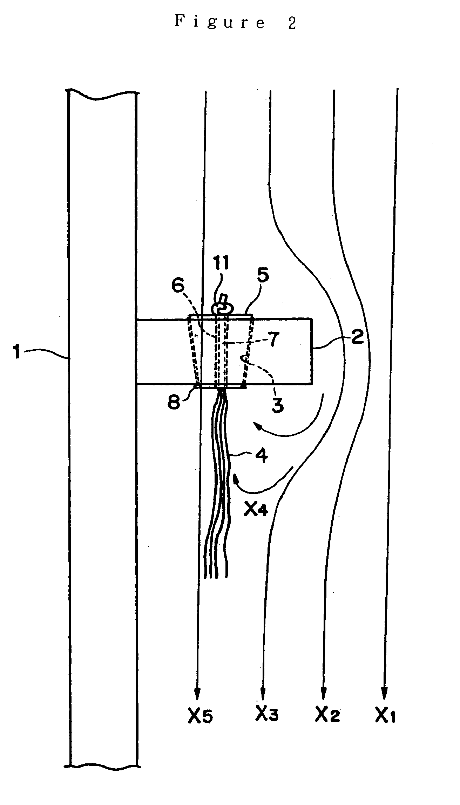

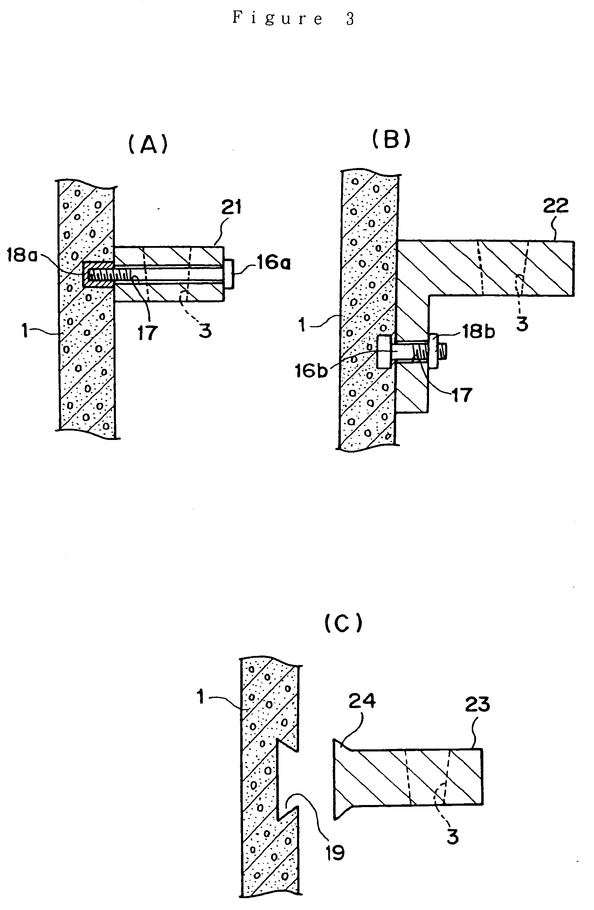

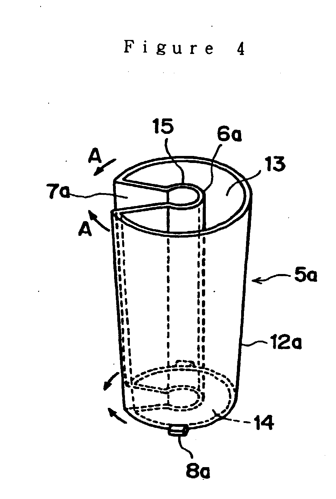

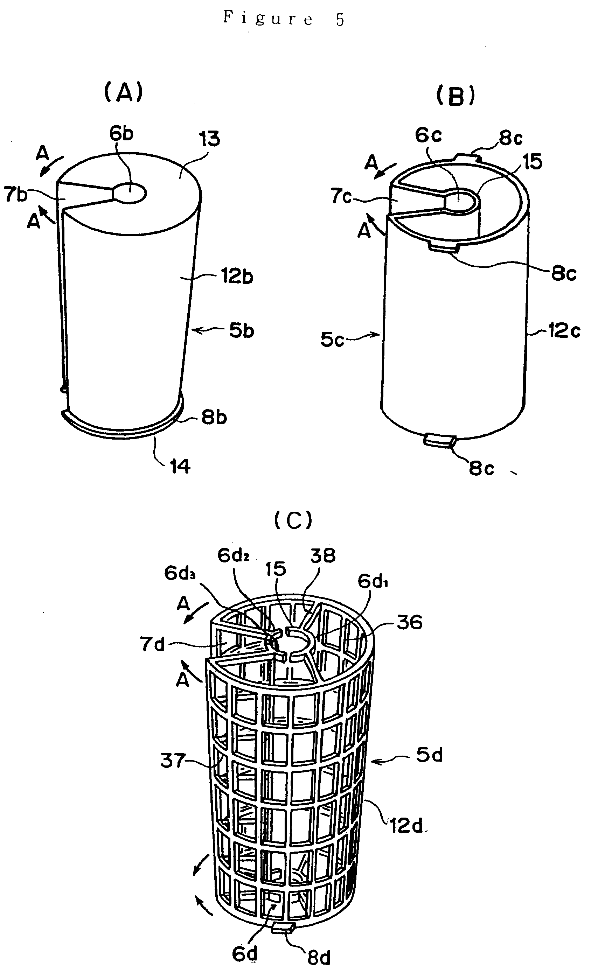

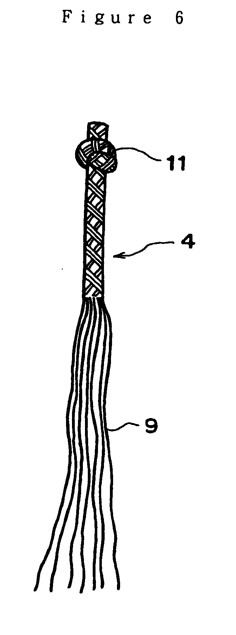

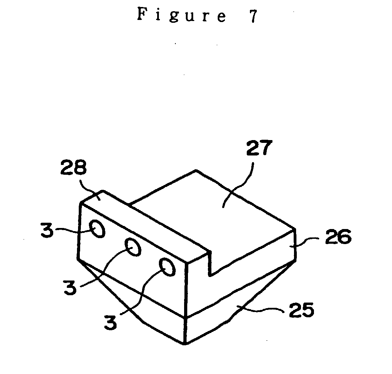

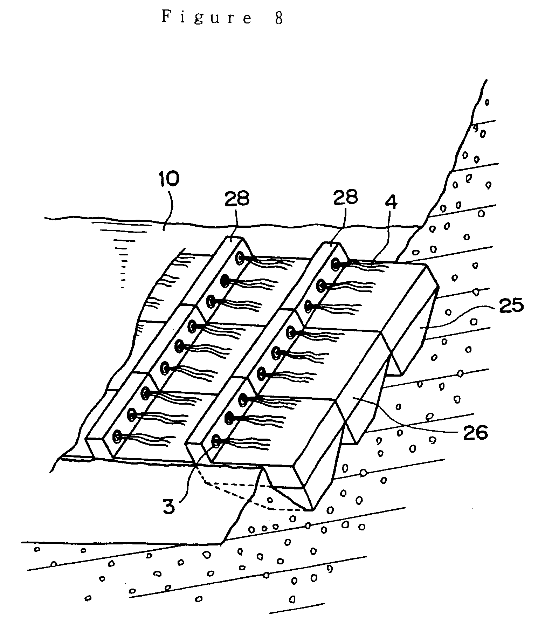

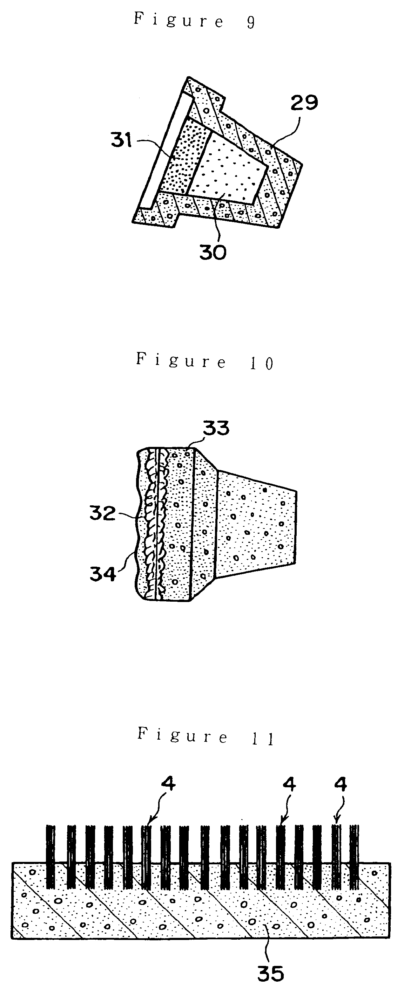

Fig. 1 is a whole view of the concrete product for afforestation (U-shaped block) which is the first embodiment of the present invention; Fig. 2 is an enlarged view of the strand planting hole part in using said embodiment; Fig. 3 is a view showing other embodiment of the bulkhead part which is a constituting part of Embodiment 1 according to the present invention, wherein (A) is a bulkhead integrated with the concrete product body by screwing, (B) is a modified example of the same, and (C) is a bulkhead engaged with the dovetailed tenon formed on the concrete product body; Fig. 4 is a whole perspective view of the stopping member used in the present embodiment; Fig. 5 (A) is a whole perspective view of the first modification of said stopping member, (B) is a whole perspective view of the second modification thereof, and (C) is a whole perspective view of the third modification thereof; Fig. 6 is a whole view of the carbon fiber strand used in this embodiment; Fig. 7 is a whole perspective view of the concrete product (square block) which is the second embodiment of the present invention; and Fig. 8 is a view showing the state of use of said embodiment. Fig. 9 is a view showing the first prior art, Fig. 10 is a view showing the second prior art, and Fig. 11 is a view showing the third prior art.

### BEST MODE FOR CARRYING OUT THE INVENTION CLAIMED

The embodiments of the present invention are described by way of examples based on the accompanying figures.

First, the concrete product for afforestations which are the embodiments of the present invention shown in the following accompanied figures are the concrete products to be used under the environment in which the water of the amount necessary for germination of seeds is supplied, e.g. U-shaped block to form water channel, square block to be used for levee of river or sea, etc. provided with green-compatible treatments.

Here, as shown in Fig. 1 which is a whole perspective view of the concrete product for afforestation of the first embodiment of the present invention and Fig. 2 which is an enlarged view of the strand planted hole portion in case of use of said embodiment, the concrete products of this embodiment comprises a concrete block body 1 formed in the vertical section of U-shape and a bulkhead 2 which projects at right angles from the inside of the side wall of the concrete block body 1. And, the respective bulkheads 2 are provided in parallel the strand planting holes 3 at equal distance in the vertical direction, and the stopping members 5 inserted in these strand planting hole 2 hold the carbon fiber strand 4.

The concrete products of this embodiment are to be used as the unit members to be used generally for constructing water channels.

Next, the bulkheads 2 are provided in projection alternately from inside the opposed side walls of the concrete block body 1. Accordingly, in water 10 which flows the water channel constructed by connecting the concrete block body 1 provided with a bulkhead 2, the water X1 for which the bulkhead 2 does not act as barrier (ref. Fig. 2) advance straightly, but the water flowing in the vicinity of the side wall of the block body 1 as shown in the arrows X2,X3,X4 in Fig. 2 is blocked by the bulkhead 2. For this reason, the water flow is changed as shown in Fig. 2 and which is formed in the periphery of the bulkhead, or, when the flow rate is large, the water splashes to take air into water, thereby increasing the dissolved oxygen amount. The number of disposition and size of the bulkheads 2 are appropriately selected to meet the conditions of water amount and the like.

Fig. 3 is a view to show other embodiment of the bulkhead part which is the first embodiment of the present invention, wherein (A) is a bulkhead which is screw-stopped to the concrete product body and integrated therewith, (B) is a modified embodiment, and (C) is a bulkhead to be engaged with the dovetailed tenon formed on the concrete product body.

The bulkheads 21 to 23 shown in Fig. 3, being different from the bulkhead 2 which is formed integrally with the concrete product body 1, are formed in separate style from the concrete product body 1.

The bulkhead 21 is previously provided with a strand planting hole 3, and a bolt hole 17 is formed in the direction crossing at right angles with the strand planting hole 3. Also, with the position agreed to the bolt hole 17, there is provided a buried nut 18a inside the side surface of the concrete product body 1. And, by inserting the bolt 16a into the bolt hole 17 and screwing into the buried nut 18a, the bulkhead 21 is integrated with the concrete product body 1.

The bulkhead 22 is formed in L-shape, and on one face thereof a strand planting hole 3 is formed, and on the other face a bolt hole 17 is provided. With the position agreed with the bolt hole 17, a bolt 16b is buried in the inside of the side surface of the concrete product body 1. In the exposed part of the bolt 16b a bolt hole 17 of the bulkhead 22 is inserted, and the projecting part is fixed with the nut 18b, by which the bulkhead 22 is integrated with the concrete product body 1.

The bulkhead 23 has a skirt 24 and a strand planting hole 3 formed at one end face opposite to the concrete product body 1. The bulkhead 23 is formed into one piece with the concrete product body 1 by engaging with the dovetailed tenon 19 formed previously on the concrete body 1.

In case of integrating any of the bulkheads 21 to 23 with the concrete product body 1, whether to fit the stopping member 5 and the carbon fiber strand 4 held by said stopping member 5 to the strand planting hole 3, or to fit the stopping member 5 and the carbon fiber strand 4 held by said stopping member 5 after integrating the bulkhead into the concrete product body 1, is appropriately selected to meet the working conditions.

As shown in Fig. 2, the strand planting hole 3 is formed in tapered cylindrical shape. The concrete block body 1 is disposed so that the wider hole side of the strand planting hole 3 is positioned on the upstream side, and the narrower hole side comes to the downstream side (ref. Fig. 2, Fig. 3).

Further, as shown in Fig. 2, in the respective strand planting hole 3 the stopping member 5 is inserted to fix by engaging the stopgap rib 8 formed on the peripheral part of the outer cylindrical part 12 of the stopping member 5 (ref. Fig. 4 and Fig. 5) with the peripheral part of the planting hole 3 of the bulkhead 2. In other words, the strand planting hole 3 is formed in the predetermined size and shape suitable for fitting said stopping member 5.

Hereinafter, referring to Fig. 4 which is the whole perspective view of the stopping member to be used in this embodiment, the structure of the stopping member 5a is explained.

The stopping member 5a has C-shaped cross section, being formed into a tapered cylindrical shape, with hollow inside. And, the stopping member 5a comprises an outer cylinder 12a having an outer diameter larger than the hole diameter of the planting hole 3 which is to be in pressure contact with the strand planting hole 3, an opening 7a formed linearly on the side peripheral surface of the outer cylinder 12a, an inner cylinder 6a which becomes the holding part of the carbon fiber strand 4, and a stopgap rib 8a provided in projection on the peripheral part of the narrower end face 14 of the outer cylinder 12a.

In this stopping member 5a, the strand planting hole 3 and the outer cylindrical pert 12 are formed in tapered shape and they have flexibility in the direction of A (ref. Fig. 4), so that when the stopping member 5a is hit into the planting hole 3 from the direction of the end face part 14, the stopgap rib 8 is engaged with the peripheral part of the planting hole 3 and fixed by adhesion. Namely, the stopping member 5a is inserted in the strand planting hole 3 with the end face 13 of the wider outer cylinder 12a set on the upstream side, and the narrower end face 14 on the downstream side, respectively.

Next, with reference to Fig. 5(A), Fig. 5(B), and Fig. 5(C), the structure of the modified examples of the stopping member 5a is explained.

In the stopping member 5b of the first modification example, a part of the side peripheral surface of the cylinder 12b formed in a solid member having tapered shape is indented in the direction of the center to form an opening 7b, and an inner cylinder 6b is formed at the central part. On the periphery of the end face 14 having a smaller caliber, a stopgap rib 8b is formed. In this stopping member 5b, in the same manner as in the stopping member 5a, by hitting into the planting hole 3, the stopgap rib 8b is engaged with the peripheral part of the planting hole 3 and fixed with stopgap.

The stopping member 5c of the second modification example has a non-tapered cylindrical outer cylinder 12c, and stopgap ribs 8c are formed on both end face peripheral parts. As the constitutions of the other parts are same as those of the stopping member 5a as described above, explanation is omitted. In case of using this stopping member 5c, the strand planting hole 3 is made to have the hole diameter slightly smaller than the stopping member 5c, and into a cylindrical shape without taper. In this modified example, because of no caliber difference in either end face, insertion in the strand planting hole 3 can be made from either direction.

In the stopping member 5d of the third modification example, a plurality of bar-like members 36 extending in vertical direction and a plurality of ring-shaped linear members 37 formed in a peripheral direction are combined in a grid form to form a net-like outer cylinder 12d. And, said outer cylinder 12d has a tapered shape, and a part of the outer cylinder 12d is recessed in the central direction to form an opening 7d. Also, at the upper and lower end parts of the outer cylinder 12d, there is formed a holding part 6d constituted by a supporting linear member 38 extending in the central direction from the ring form linear member 37, an integrally formed C-shaped large curved part 6d1, and small curved parts 6d2, 6d3 opposed thereto. This holding part 6d holds the carbon fiber strand 4 introduced from the opening part 7d. The small curved parts 6d2, 6d2 formed at the upper and lower end faces are mutually connected through the bar-like linear member 38 for reinforcement.

As the insides of the stopping members 5a, 5c and 5d excluding the above stopping member 5b have spaces, they form the channels for water X5 (ref. Fig. 2) directed to the carbon fiber strand 4 exposed to the downstream side.

Here, as the stopping members 5a, 5b, 5c and 5d are formed of the plastic materials having flexibility, the opening 7 formed on the side peripheral surface of the outer cylindrical part 12 is blocked when force is exerted in the direction of the arrow A as shown in Fig. 4 and Fig. 5. By this action, the stopping member 5 becomes smaller in size than the hole diameter of the strand planting hole 3, and can be inserted in the strand planting hole 3. And, the stopping member 5 inserted in the strand planting hole 3 becomes released state, and by its restoring force the side peripheral part of the outer cylindrical part 12 is brought into pressure contact with the inner peripheral surface of the strand planting hole 3. Further, by the stoppage of the stopgap rib 8 provided in projection on the front and rear peripheral parts of the outer cylinder 12 at the peripheral part of the strand planting hole 3, the stopping member 5 is set into the strand planting hole 3 and fixed.

Hereinafter, with respect to the carbon fiber strand to be used in this embodiment, explanation is made with reference to Fig. 6 which is the whole view thereof.

The carbon fiber strand 4 is a member formed by the collection of plural carbon fibers 9. The use of the carbon fiber 9 as a material for this fiber is because said fiber is soft and flexible, and excellent in adsorption of plants and microorganisms. The number, length, width, etc. of the carbon fiber 9 to constitute the carbon fiber strand 4 are appropriately selected depending on the installation conditions of concrete blocks including water volume, flow rate, etc.

One end part of the carbon fiber strand 4 is in a knitted condition, and by fastening this part there is formed a stopping protuberance 11. As shown in Fig. 2, this stopping protuberance 11 plays the role of hooking the carbon fiber strand 4 on the stopping member 5 by engaging the carbon fiber strand 4 of the stopping member 5 with the end of the inner cylinders 6a - 6c or holding part 6d (ref. Fig. 2, Fig. 4). Namely, the stopping protuberance 11 prevents slipping off of the carbon fiber strand 4 from the stopping member 5 by water flow.

Instead of the stopping protuberance 11, a clamp member such as insulock may be used to prevent sliding off of the carbon fiber strand 4.

Here, the carbon fiber strand 4 is introduced at the end part incorporated with the side having the stopping protuberance 11 from the opening 7 (ref. Fig. 4, Fig. 5) of the stopping member 5 and held by the inner cylinders 6a - 6c formed near the center of the stopping member 5 or the holding part 6d and made into one piece with the stopping member 5.

On the other hand, the other end part in which no carbon fiber strand 4 is knit is exposed from the end face disposed on the downstream side of the stopping member 5 (ref. Fig. 1, Fig. 2). The exposed part of the carbon fiber strand 4 floats more vigorously in water to play the role of waterweed by the water passing through the inside of the hollow stopping members 5a, 5c, 5d (ref. Fig. 2 X5) and water flow which forms vortex (ref. Fig. 2 X4), and the like.

Next, Fig. 7 is a whole perspective view of the concrete product according to the second embodiment of the present invention. Fig. 8 is a view to show the state of use of said embodiment.

The square block according to this embodiment is a concrete product for forming levee walls by arranging in parallel square blocks in all directions by burying the submerging part 25 in the coast or bank of river or seaside, with said product furnished with green plant growing.

As shown in Fig. 7 and Fig. 8, the present embodiment has a burying part 25 of nearly truncated pyramid shape placed upside down, with an upright wall 28 projecting upward from the top face 27 of the square face part 26 formed on the upper part of the burying part 25.

On the upright wall 28, there are provided the strand planting holes 3 in parallel in the right and left directions, and on each of said strand planting holes 3 the carbon fiber strands 4 carried by the stopping member 5 are planted. The upright wall 28 projects into water to function to change the current and increase the dissolved oxygen amount in water.

By using this square block, green planting can be simply provided to the extensive levee walls.

The number of the carbon fiber strands 4 to be planted on the square blocks may be adjusted appropriately to meet the conditions of layout such as the water volume, flow rate, and the like.

### INDUSTRIAL APPLICABILITY

The effects obtainable by the typical ones of the inventions disclosed by this application are briefly explained as follows:

As there is adopted not such a structure as to plant a carbon fiber strand in concrete in advance and fix them in one piece but a structure to set the stopping member holding the carbon fiber strands into the strand planting hole of the concrete product in freely detachable manner, re-planting can be easily achieved at a later stage. Furthermore, the replacement work of carbon fiber strands can be easily performed, as it is not necessary to carry out the work in the unit of the concrete block.

And, as it is possible to select appropriately the density and the kind of the carbon fiber strands to be planted so as to meet the environmental conditions such as water amount and flow rate, the application range is extensive.

Moreover, due to the formation of bulkhead and upright wall, the water flow can be directed to zigzag form or changed, so that the dissolved oxygen amount in the surrounding aqueous zone increases to accelerate the settlement of the microorganisms and aquatic plants on the carbon fiber strands. In other words, green growth on the concrete products is accelerated.

Accordingly, the present invention is suitable for the concrete product for afforestations which are minutely adaptable to the environmental conditions such as water amount, flow rate, water quality, plantation, of the place of installation.

## Claims

1. A concrete product for afforestation comprising carbon fiber strands planted in the predetermined position on the outer surface of the concrete product body which is to be used under the environment in which sufficient amount of water necessary for germination of seeds is supplied,
said concrete product body being provided with strand planting holes, and and end of said carbon fiber strand being held by the flexible stopping member having a cross-sectional shape of C including an opening for introducing the strand on the side in which plant is inserted and fixed.

2. A concrete product for afforestation according to claim 1, wherein said stopping member comprises an outer cylindrical part elastically engaging a strand planting hole and an inner cylindrical part which is a strand holding part formed in one piece.

3. A concrete product for afforestation according to claim 2, wherein the above strand planting hole is formed in tapered shape with the smaller diameter toward the exposed side of the carbon fiber strand,
the outer cylindrical part of said stopping member is formed in a tapered shape to meet the strand planting hole, and a stop-gap rib to be engaged with the peripheral part of the strand planting hole is formed at the tip of the outer cylindrical part.

4. A concrete product for afforestation according to any one of claims 1 to 3, wherein said concrete product has U-shaped grooves, and inside the side wall of the U-shaped groove body there are provided in projection the bulkheads for flowing water in zigzag directions right and left alternately, and a strand planting hole is provided on the respective bulkhead.

5. A concrete product for afforestation according to any one of claims 1 to 3, wherein said concrete product is a square faced block, an upright wall is projected on the front face of the square block body, and a strand planting hole is provided on said upright wall.
